# EUROPÄISCHE PATENTSCHRIFT

(11) **EP 4 058 418 B1**
(45) Veröffentlichungstag und Bekanntmachung des Hinweises auf die Patenterteilung: **24.07.2024**
(21) Anmeldenummer: 20804254.9
(22) Anmeldetag: 10.11.2020
(51) Int. Cl.: C03C 27/06

(54) **ZWEITEILIGE REAKTIONSGEFÄSSE AUS GLAS, HERSTELLUNGSVERFAHREN UND VERFAHREN ZU ANALYSE**
TWO-PART REACTION VESSELS MADE OF GLASS, PRODUCTION METHOD AND ANALYTICAL METHOD
RÉCIPIENTS DE RÉACTION EN DEUX PARTIES CONSTITUÉS DE VERRE, PROCÉDÉ DE FABRICATION ET PROCÉDÉ ANALYTIQUE

(30) Priorität: 12.11.2019 DE 102019217466
(43) Veröffentlichungstag der Anmeldung: 21.09.2022
(73) Patentinhaber: LPKF Laser & Electronics SE, 30827 Garbsen (DE)
(72) Erfinder: KRÜGER, Robin, 30419 Hannover (DE); SCHULZ-RUHTENBERG, Malte, 31515 Wunstorf (DE); VAN AALST, Jan, 30827 Garbsen (DE); WOLLER, Moritz, 31535 Neustadt (DE)
(74) Vertreter: Taruttis, Stefan Georg
(86) Internationale Anmeldenummer: PCT/EP2020/081592
(87) Internationale Veröffentlichungsnummer: WO 2021/094290

(56) Entgegenhaltungen:
- EP-A1- 1 867 612
- JP-A- 2005 351 774
- US-A1- 2005 142 812
- US-A1- 2009 013 724
- US-A1- 2017 352 553

## Beschreibung

Die vorliegende Erfindung betrifft ein Verfahren zur Herstellung von Reaktionsgefäßen aus Glas, die in Form von Ausnehmungen in Glas geformt sind. Das Glas, in dem die Reaktionsgefäße in einer Anordnung eingeformt sind, besteht aus zwei oder mehr als zwei miteinander verbundenen Glaselementen, die optional unmittelbar miteinander verbunden sind oder mittels einer zwischen den Glaselementen angeordneten Verbindungsschicht. Das Verfahren erzeugt eine Anordnung einer Vielzahl von Reaktionsgefäßen, die in Glas eingeformt ist, z.B. eine Anordnung von 25 x 25 Feldern von jeweils 8 x 12 Reaktionsgefäßen. Die Reaktionsgefäße sind in einer Vielzahl in einer Glasplatte ausgebildet, die aus einer ersten und einer damit dicht verbundenen zweiten Glasplatte oder weiteren in gleicher Weise verbundenen Glasplatten bestehen kann.

Das Verfahren hat den Vorteil, ohne mechanische Einwirkung auf ein massives Glas Ausnehmungen zu formen, die Reaktionsgefäße bilden, die daher keine mechanischen Beschädigungen aufweisen, z.B. keine Mikrorisse. Die Reaktionsgefäße weisen ein großes Streckungsverhältnis von Tiefe zu Durchmesser auf. Ein weiterer Vorteil liegt darin, dass das Verfahren zur Herstellung der Reaktionsgefäße zumindest ohne selektive Beschichtung der Glasoberfläche ablaufen kann, in der die Querschnittsöffnungen der Reaktionsgefäße gebildet sind, optional ohne jede Beschichtung der Oberflächen des Glases, in dem die Reaktionsgefäße gebildet werden.

### Stand der Technik

Die JP 2005351774 A beschreibt ausweislich der Computerübersetzung ein Verfahren zur Herstellung eines Dosierkopfs, mit dem Flüssigkeit auf ein Mikroarray getropft werden kann. In einer ersten Platte hält eine konisch zulaufende Durchbrechung eine Kugel vor einer Auslassöffnung, mit einer an der ersten Platte angebrachten zweiten Platte, die einen in der Durchbrechung mündenden Flüssigkeitskanal enthält.

Die US 2005/0142812 A1 beschreibt die Herstellung eines Dosierkopfs, der einen Glasträger mit einem durch Laserbestrahlung und Ätzen erzeugten Durchgangsloch sowie einer Ausnehmung mit einer darin angeordneten Leiterbahn und einer Halbleiterplatte, die an der Oberfläche des Glasträgers anliegt, die die Ausnehmung mit der Leiterbahn aufweist. Das Ätzen des Durchgangslochs durch den Glasträger erfolgt nach dem Anbringen der Halbleiterplatte am Glasträger. Anstelle der Halbleiterplatte kann ein anderes Material mit dem Glasträger verbunden werden, solange dies andere physikalische Eigenschaften als der Glasträger hat.

Die US 2017/0352553 A1 beschreibt das Ätzen von Vias in eine Glas- oder Keramikunterlage durch Ätzen, wenn diese mit einer Beschichtung maskiert ist, mit anschließendem Entfernen des Beschichtungsmaterials. Optional kann ein Träger an die Oberfläche der Unterlage gebondet werden, die mit dem Beschichtungsmaterial maskiert war und im Anschluß an das Bonden kann der Träger von der Unterlage, die die geätzten Vias enthält, entfernt werden, da die Verbindung zwischen Unterlage und Träger durch das Bonden reversibel ist.

Die US 2009/0013724 A1 beschreibt das Ätzen von Durchgangslöchern in eine Glasplatte oder von zylindrischen Sacklöchern.

Deutsch et al., Lab Chip, 2006, 69, 995-1000, beschreiben die Herstellung von Reaktionsgefäßen eines Durchmessers von 20 µm bei 8 µm Tiefe in einer Glasplatte durch Ätzen nach dem Auftragen einer Maske aus Chrom und darauf Photoresist.

Die US 2003/0211014 A1 beschreibt die Herstellung von Reaktionsgefäßen in Glas mittels eines mit Ultraschall beaufschlagten Werkzeugs und Schleifmittel zwischen dem Werkzeug und dem Glas.

Die EP 1 867 612 A1 beschreibt Mikrotiterplatten mit 96 Näpfen aus einem Boden aus Glas, das für UV durchlässig ist und durch Glasfritte mit einer Glasplatte verbunden ist, in der durchgehende Ausnehmungen die Seitenwände der Näpfe bilden.

Die EP 2 011 857 A1 beschreibt das Erzeugen von Oberflächenstrukturen am Boden von Mikrotiterplatten mittels eines photolithographischen Verfahrens.

### Aufgabe der Erfindung

Der Erfindung stellt sich die Aufgabe, ein alternatives Herstellungsverfahren bereitzustellen, das insbesondere geeignet ist, Reaktionsgefäße mit einem großen Streckungsverhältnis bei insgesamt kleinem Volumen in Glas herzustellen, sowie eine Anordnung einer Vielzahl solcher Reaktionsgefäße in Glas bereitzustellen. Bevorzugt soll das Verfahren geeignet sein, eine Anordnung solcher Reaktionsgefäße bereitzustellen, bei der das Glas bei Bestrahlung mit Licht einen hohen optischen Kontrast zu Zellen in den Reaktionsgefäßen bildet.

### Beschreibung der Erfindung

Die Erfindung löst die Aufgabe mit den Merkmalen der Ansprüche 1 und 12 und insbesondere mit einem Verfahren zur Herstellung von Reaktionsgefäßen aus Glas sowie den mit dem Verfahren erhältlichen Reaktionsgefäßen aus Glas. Das Verfahren weist die Schritte
1. Einstrahlen, bevorzugt punktförmiges Einstrahlen eines Laserstrahls einer Wellenlänge, für die die erste Glasplatte durchlässig ist, auf die Stellen der Oberfläche einer ersten Glasplatte, an denen jeweils eine Ausnehmung als Reaktionsgefäß erzeugt werden soll,
2. Ätzen der ersten Glasplatte, bevorzugt für eine Zeitdauer, die zum Erzeugen von Ausnehmungen einer Tiefe, bevorzugt von zumindest 40 µm oder zumindest 30 µm, bevorzugt mit einem Streckungsverhältnis von zumindest 2, zumindest 4, zumindest 5 oder zumindest 6 von Tiefe zu Durchmesser, der in der Ebene der ersten Oberfläche gemessen ist, entlang der Stellen ausreicht, um Ausnehmungen zu erzeugen, die sich über die vollständige Dicke der ersten Glasplatte erstrecken,
3. nach Ätzen der ersten Glasplatte zur Ausbildung von Ausnehmungen, Verbinden einer zweiten Platte mit einer Oberfläche der ersten Glasplatte,
auf oder besteht daraus.

Jede Glasplatte kann hierbei ein Gegenstand aus Glas mit einer lateralen Ausdehnung sein, die größer ist als die Dicke. Glasplatten können also einen rechteckigen, runden oder anders geformten Umfang aufweisen, der ihre gegenüberliegenden Oberflächen begrenzt.

Die Reaktionsgefäße werden durch Ausnehmungen in einer ersten Glasplatte gebildet, wobei die Ausnehmungen genau eine Öffnung aufweisen, die in der Ebene einer ersten Oberfläche der ersten Glasplatte liegt. Anwendungen der Reaktionsgefäße sind nicht auf chemische Reaktionen beschränkt, sondern schließen biochemische, biologische und physikalische Prozesse ein. Dies können Prozesse mit einzelnen Zellen, die tierische oder pflanzliche Zellen oder Hefezellen sein können, Bakterien, Viren, Proteinen etc. oder mit Clustern dieser sein.

Das punktförmige Einstrahlen wird durch Fokussieren der Laserstrahlung auf einen Punkt mit einer Größe von wenigen Mikrometern, z.B. 1 bis 10 µm oder bis 5 µm, erzielt. Dabei ist es vorteilhaft, wenn der Fokus der Laserstrahlung sich über eine Länge entlang der Ausbreitungsrichtung des Laserstrahls erstreckt, die wesentlich größer ist als die Rayleigh-Länge eines entsprechenden Laserstrahls mit Gauß-Profil. Dies kann durch geeignete optische Einrichtungen, z.B. diffraktiv-optische Elemente, erreicht werden. Das Einstrahlen kann ein Durchstrahlen sein, oder ein Einstrahlen weniger tief bis in einen an die erste Oberfläche angrenzenden ersten Dickenabschnitt der ersten Glasplatte erfolgen, z.B. dadurch dass sich der Fokus der Laserstrahlung in Richtung der Ausbreitungsrichtung der Laserstrahlung nicht über die gesamte Dicke der Glasplatte erstreckt. Da eine Wechselwirkung zwischen Laserstrahlung und Material der Glasplatte nur im Fokus stattfindet, ist es so möglich, den Wechselwirkungsbereich innerhalb der ersten Glasplatte enden zu lassen. Bevorzugt besteht die Laserstrahlung aus Laserpulsen.

Dabei kann beim Verbinden einer zweiten Glasplatte mit einer Oberfläche der ersten Glasplatte die Oberfläche der ersten Glasplatte die erste Oberfläche der ersten Glasplatte sein, auf die der Laserstrahl eingestrahlt wurde, oder die dieser ersten Oberfläche gegenüberliegende zweite Oberfläche.

Die erste Oberfläche der ersten Glasplatte, sowie in Abwesenheit einer Beschichtung aus Ätzresist auch die dieser gegenüberliegende zweite Oberfläche, wird beim Ätzen an den Stellen, auf die der Laser auf die erste Glasplatte eingestrahlt wurde und an denen der Laserstrahl gegenüber ausgetreten ist, deutlich schneller abgetragen, als die benachbarten Bereiche. Die Bereiche der ersten Oberfläche und ggf. der zweiten Oberfläche der ersten Glasplatte werden daher wegen der Abwesenheit einer Beschichtung, z.B. aus Ätzresist, im Abstand von den Stellen der punktförmigen Laserbestrahlung langsamer und gleichförmig abgetragen. Daher wird die erste Oberfläche mit Ausnahme der Ausnehmungen von Oberflächenabschnitten gebildet, die in einer Ebene angeordnet sind, aus der sich die Ausnehmungen in das Glasvolumen der ersten Glasplatte erstrecken. Die Oberflächenabschnitte, die in einer gemeinsamen Ebene angeordnet sind und die erste Oberfläche bilden, von der die Ausnehmungen ausgenommen sind, werden von den Stirnflächen der Wandungen gebildet, die zwischen den Ausnehmungen liegen. Bei Abwesenheit einer Beschichtung von der der ersten Oberfläche gegenüberliegenden zweiten Oberfläche können sich die Ausnehmungen auch ausgehend von der zweiten Oberfläche entlang der Stellen, an denen der Laserstrahl punktförmig eingestrahlt bzw. durchgestrahlt wurde, in das Glasvolumen erstrecken. Dabei können Ausnehmungen gebildet werden, die einen sanduhrförmigen Längsschnitt durch die Dicke der Glasplatte aufweisen.

In Schritt 2 erfolgt das Ätzen für eine Zeitdauer, die zur Ausbildung von Ausnehmungen ausreicht, die sich über die vollständige Dicke der ersten Glasplatte erstrecken, so dass die Tiefe der Ausnehmungen der Dicke der ersten Glasplatte entspricht.

Die in Schritt 1 punktförmig eingestrahlten Laserstrahlen, an denen in Schritt 2 eine Ausnehmung, die ein Reaktionsgefäß bilden soll, geätzt wird, sind in der Ebene der ersten Oberfläche der ersten Glasplatte in einem Abstand von zumindest oder genau dem Durchmesser eines der Reaktionsgefäße zuzüglich der Dicke einer Wand zwischen den Reaktionsgefäßen angeordnet. Der Durchmesser der Reaktionsgefäße ist durch die Reaktionsbedingungen und die Dauer des Ätzens einstellbar, da das Ätzen konzentrisch um den linearen Pfad erfolgt, den der eingestrahlte Laserstrahl durch die erste Glasplatte genommen hat. Die Wände, die zwischen den Reaktionsgefäßen angeordnet sind, enden in einer gemeinsamen Ebene. Die Endflächen dieser Wände liegen in einer gemeinsamen Ebene und bilden die erste Oberfläche bzw. bilden in einer gemeinsamen Ebene die erste Oberfläche, wobei die erste Oberfläche von den Ausnehmungen unterbrochen ist. Die erste Oberfläche wird bevorzugt nur von den Querschnitten der Ausnehmungen unterbrochen.

Der Laserstrahl ist an jeder der Stellen, an denen er auf die erste Glasplatte eingestrahlt wird, gepulst, z.B. mit einer Wellenlänge von 1064 nm, bevorzugt mit Pulslängen von maximal 100 ps oder maximal 50 ps, bevorzugt maximal 10 ps. Generell ist der Laser eingerichtet, dass der Laserstrahl zwischen den Stellen nicht auf die erste Glasplatte trifft. Der Laserstrahl wird punktförmig und senkrecht auf die Oberfläche der ersten Glasplatte eingestrahlt. Bevorzugt bildet diese Oberfläche, auf die der erste Laserstrahl eingestrahlt wurde, die erste Oberfläche der ersten Glasplatte.

Das Ätzen erfolgt z.B. mit Flusssäure, z.B. 1 bis 48 Gew.-%, und/oder Schwefelsäure und/oder Salzsäure und/oder Phosphorsäure und/oder Salpetersäure, oder Kalilauge, bei z.B. bis zu 140°C.

Die erste Glasplatte kann z.B. eine Dicke vor dem Ätzen von bis zu 1000 µm, bevorzugt 100 bis 1000 µm, z.B. bis 800 µm, z.B. 300 bis 500 µm aufweisen, nach dem Ätzen eine um 50 bis 700 µm geringere Dicke, z.B. eine bis 200 µm geringere Dicke.

Die Ausnehmungen erstrecken sich bevorzugt in einem Winkel von z.B. 0° bis 15° kegel- oder kegelstumpfförmig zulaufend und von der Oberfläche der ersten Glasplatte ausgehend in deren Volumen.

Dabei liegt die zweite Oberfläche der ersten Glasplatte ihrer ersten Oberfläche gegenüber, auf die der Laserstrahl gerichtet war, bzw. durch die der Laserstrahl in die erste Glasplatte eingewirkt hat.

Generell wird die erste Glasplatte ohne eine Beschichtung, z.B. ohne Maske und/oder ohne Ätzresist, dem Ätzen unterzogen, so dass das Verfahren den Vorteil hat, ohne Auftragen und ohne Entfernen von Ätzresist von einer Glasplatte durchgeführt zu werden. Generell bleibt die erste Oberfläche der ersten Glasplatte ohne Ätzresist und ohne Maske und wird ohne Ätzresist geätzt.

Generell optional kann jede Stelle der ersten Glasplatte, an denen eine Ausnehmung erzeugt werden soll, an mehreren voneinander beabstandeten Positionen, z.B. an zumindest 3 oder zumindest 10 oder zumindest 30 Positionen, mit Laserstrahlen punktförmig bestrahlt, wobei die Laserstrahlen bevorzugt parallel zueinander und senkrecht auf die erste Glasplatte eingestrahlt werden, nacheinander oder gleichzeitig. Die Positionen bilden die Stelle, an der das Ätzen die erste Glasplatte schneller abträgt, als an davon entfernten Oberflächenbereichen. Die Positionen, an denen der Laser eingestrahlt wurde, führen beim Ätzen zu einem gleichmäßig schnellen Abtrag des Glases und bilden gemeinsam eine Ausnehmung. Die Positionen, die im Bereich einer Stelle eingestrahlt werden und eine Stelle bilden, sind z.B. in einem Abstand von 1 bis 10 µm angeordnet. Bevorzugt sind die Positionen innerhalb des Bereichs um jede Stelle angeordnet, in dem jeweils eine Ausnehmung gebildet werden soll. Bevorzugt sind die Positionen, an denen Laserstrahlen um eine Stelle oder zur Ausbildung einer Stelle eingestrahlt werden, in einem Abstand von 1 bis 10 µm, z.B. 2 bis 5 µm oder bis 3 µm, der insbesondere in der Ebene der ersten Oberfläche der ersten Glasplatte bestimmt ist.

Generell kann eine Ausnehmung durch einen einzelnen Laserpuls oder mehrere Laserpulse erzeugt werden. Bei einem einzelnen Laserpuls wird der Durchmesser der Ausnehmung vornehmlich durch die Ätzdauer bestimmt. Bei der Erzeugung einer Ausnehmung mit mehreren Laserpulsen wird der Durchmesser der Ausnehmung von Anzahl und Abstand der Positionen bestimmt, an denen Laserstrahlen für eine Stelle eingestrahlt werden und in die erste Glasplatte eindringen. Die Tiefe der Ausnehmung in das Volumen der ersten Glasplatte kann durch die Zeitdauer des Ätzens bestimmt werden und dadurch, dass der Laserstrahl nur zu einem Anteil in die erste Glasplatte eindringt bzw. die erste Glasplatte nicht vollständig durchstrahlt.

Optional wird um jede Stelle, an der eine Ausnehmung gebildet werden soll, auf einem umfänglich geschlossenen Weg, der bevorzugt ringförmig, rechteckig oder hexagonal ist, ein Laserstrahl eingestrahlt, der z.B. durch nebeneinander eingestrahlte Laserstrahlpulse gebildet wird. Dabei können Laserstrahlpulse auf dem umfänglich geschlossenen Weg auf die erste Glasoberfläche eingestrahlt werden, z.B. in einem auf der ersten Oberfläche der ersten Glasplatte bestimmten Abstand der Laserstrahlpulse von 3 µm nebeneinander. Optional kann daher die erste Glasplatte an jeder Stelle an mehreren voneinander beabstandeten Positionen mit Laserstrahlen jeweils punktförmig bestrahlt werden und um diese Positionen herum ein Laserstrahl, z.B. durch nebeneinander eingestrahlte Laserstrahlpulse gebildet, entlang eines umfänglich geschlossenen Wegs eingestrahlt werden. Das Einstrahlen eines Laserstrahls entlang eines umfänglich geschlossenen Wegs hat den Vorteil, dass beim anschließenden Ätzen Ausnehmungen mit einer Wandung gebildet werden, die sich von der ersten Oberfläche erstreckt und einen Querschnitt aufweist, der den umfänglich geschlossenen Weg einschließt.

Generell können Laserpulse an Positionen, die eine Stelle bilden, an der durch Ätzen eine Ausnehmung gebildet wird, bis in unterschiedliche Tiefen in die erste Glasplatte eingestrahlt werden. So können z.B. Laserpulse an Positionen tiefer in die Dicke der ersten Glasplatte eingestrahlt werden und tiefer eindringen und andere Laserpulse an Positionen weniger tief in die Dicke der ersten Glasplatte eingestrahlt werden. Beim anschließenden Ätzen werden an den Positionen, an denen Laserpulse tiefer in die Glasplatte eingestrahlt wurden, tiefere bzw. weitere Ausnehmungen gebildet, und an den Positionen, an denen Laserpulse weniger tief in die Glasplatte eingestrahlt wurden, wird der Boden der Ausnehmung in geringerer Tiefe gebildet. Generell kann, abhängig vom Abstand der Positionen, an jeder Position eine konkave Vertiefung im Boden gebildet werden. Eine Ausnehmung, die einen Boden und darin weitere tiefere Ausnehmungen aufweist, kann durch Einstrahlen von Laserpulsen in dem Teil der Positionen, die den Boden bilden sollen, bis weniger tief in die erste Glasplatte und Einstrahlen von Laserpulsen in dem Teil der Positionen, die weitere Ausnehmungen, die sich ausgehend von dem Boden tiefer in die erste Glasplatte erstrecken sollen, bis tiefer in die erste Glasplatte und anschließendes Ätzen hergestellt werden. Für einen größeren Durchmesser weiterer Ausnehmungen, die sich ausgehend vom Boden einer Ausnehmung tiefer in die erste Glasplatte erstrecken, können Laserpulse, die tiefer in die erste Glasplatte eingestrahlt werden, an benachbarten Positionen, z.B. in einem Abstand von 1 bis 10 µm, z.B. 2 bis 5 oder bis 3 µm angeordnet sein, so dass an diesen Positionen das Ätzen tiefer in die erste Glasplatte eindringt. So können an einem Teil der Positionen Laserpulse weniger tief in die erste Glasplatte eingestrahlt werden, und an einem Teil der Positionen Laserpulse tiefer in die erste Glasplatte eingestrahlt werden, so dass beim Ätzen an den Positionen, an denen die Laserpulse weniger tief eingestrahlt wurden, ein Boden mit konkaven Vertiefungen in geringerer Tiefe erzeugt wird und an den Positionen, an denen Laserpulse tiefer eingestrahlt wurden, weitere Ausnehmungen erzeugt werden, die sich tiefer in die erste Glasplatte erstrecken.

In einer weiteren Ausführungsform z.B. einem Verfahren, das aus den Schritten 1 bis 3 besteht, werden die Reaktionsgefäße von Ausnehmungen gebildet, die sich durch die vollständige Dicke der ersten Glasplatte erstrecken, wobei eine zweite Platte mit einer Oberfläche der ersten Glasplatte verbunden ist. Die zweite Platte bildet den Boden der Reaktionsgefäße. Dabei kann die zweite Platte mit der ersten Oberfläche, bevorzugt mit der zweiten Oberfläche der ersten Glasplatte verbunden sein. Die Anordnung der zweiten Platte an der zweiten Oberfläche der ersten Glasplatte bildet Reaktionsgefäße aus, die von ihrer Öffnung, die in der Ebene der ersten Oberfläche der ersten Glasplatte liegt, kegelförmig zur zweiten Glasplatte zulaufen und daher optional keine Hinterschneidung bilden. Die zweite Platte ist bevorzugt eine zweite Glasplatte und wird daher stellvertretend für zweite Platten aus anderem Material wahlweise generell auch als zweite Glasplatte bezeichnet. Die zweite Platte kann eine Glasplatte sein oder Silizium, Saphir, Keramik, Metall oder zumindest zwei Schichten aus diesen aufweisen oder daraus bestehen.

Die Reaktionsgefäße weisen z.B. eine Tiefe von zumindest 40 µm, zumindest 50 µm oder zumindest 100 µm oder zumindest 150 µm auf, z.B. bis 250 µm oder bis 200 µm. Die Reaktionsgefäße weisen z.B. einen Durchmesser von zumindest 10 µm oder zumindest 30 µm auf, z.B. bis 200 µm oder bis 1 mm, generell bevorzugt mit eine Streckungsverhältnis von Tiefe zu Durchmesser von zumindest 2, zumindest 4, zumindest 5 oder zumindest 6 auf. Die Ausnehmungen der ersten Glasplatte haben z.B. ein Innenvolumen innerhalb der ersten Glasplatte von 1 pL bis 1 µL.

Das Verbinden der zweiten Platte, die insbesondere eine zweite Glasplatte ist, mit der ersten Glasplatte, bevorzugt mit der zweiten Oberfläche der ersten Glasplatte, kann durch Aufeinanderlegen der ersten und zweiten Platten unmittelbar aufeinander mit anschließendem Erwärmen erfolgen, z.B. bei Quarzglas auf 400 bis 1200 °C. Dabei kann das Verbinden bei längerer Dauer und niedrigerer Temperatur, bzw. höherer Temperatur und kürzerer Dauer des Verbindungsprozesses erfolgen, wobei die Temperatur von der Maximaltemperatur abhängt, bei der die erste Glasplatte und die zweite Platte noch ausreichend formstabil sind.

Alternativ kann die zweite Glasplatte durch anodisches Bonding mit der zweiten Oberfläche der ersten Glasplatte verbunden werden. Dabei wird die zweite Oberfläche der ersten Glasplatte und/oder die der ersten Glasplatte zugewandte Oberfläche der zweiten Glasplatte mit Silizium beschichtet, z.B. mittels Kathodenzerstäubung, auch Sputtern genannt, die erste Glasplatte wird an der zweiten Platte angeordnet und es wird eine Spannung von, z.B.300 - 500 V, angelegt und optional erwärmt, z.B. auf 400 °C. Dadurch werden die beiden Glasplatten mittels Diffusion von im Glas enthaltenen Ionen (z.B. Na, K) und Sauerstoffanionen miteinander verbunden.

Alternativ können die Glasplatten durch Fusion-Bonding vorzugsweise ohne Zwischenschicht durch Inkontaktbringen einer der Oberflächen der ersten Glasplatte mit einer der Oberflächen der zweiten Glasplatte und Erwärmen und Druckausüben verbunden werden.

Die gleichen Verbindungsverfahren oder Kombinationen der Verfahren können genutzt werden, um weitere Glasplatten mit der ersten und/oder zweiten Glasplatte zu verbinden.

Generell kann das Erwärmen in einem Ofen erfolgen.

Weiter generell kann die zweite Glasplatte aus Glas bestehen, das für eine chemische Oberflächenmodifizierung reaktiver ist als das Glas der ersten Glasplatte. Z.B. kann die zweite Glasplatte aus Kalk-Natron-Glas bestehen, die erste Glasplatte aus Borosilikatglas, so dass in die Reaktionsgefäße eingefüllte wässrige oder organische Reagenzien vornehmlich an das Glas der zweiten Glasplatte binden, die den Boden der Reaktionsgefäße bildet. Zusätzlich oder alternativ kann die zweite Glasplatte aus einer anderen Glaszusammensetzung bestehen als die erste Glasplatte, z.B. die zweite Glasplatte aus Quarzglas oder Fused Silica, die erste Glasplatte aus Borosilikatglas.

Optional können auf die Oberfläche einer zweiten Platte, die mit einer ersten Glasplatte verbunden wird, Leiterbahnen aufgebracht sein, z.B. Leiterbahnen, die getrennt nebeneinander angeordnet sind und die mit getrennten Anschlussflächen verbunden sind und daher voneinander getrennt kontaktierbar sind. Die erste Leiterbahn bedeckt bevorzugt zumindest einen Abschnitt der Ausnehmungen, die in der ersten Glasplatte erzeugt sind, optional überdeckt die erste Leiterbahn die erste Oberfläche vollflächig und zumindest einen Anteil der Ausnehmungen. Die zweite Leiterbahn verläuft entlang des Bereichs, der den Boden eines Reaktionsgefäßes bildet und ist bevorzugt mit einer Kontaktfläche verbunden, die auf einem Abschnitt der zweiten Glasplatte angeordnet ist, der die erste Glasplatte überragt. Die erste und die zweite Leiterbahn können durch Aufsputtern oder Drucken aufgebracht werden, die erste Leiterbahn z.B. nach dem Ätzen, und die zweite Leiterbahn vor dem Verbinden der zweiten Glasplatte mit der ersten Glasplatte. Optional können in der zweiten Glasplatte Vertiefungen gebildet sein, in denen die zweiten Leiterbahnen angeordnet sind. Solche Vertiefungen können entlang der Oberfläche der zweiten Glasplatte verlaufen, die der ersten Glasplatte zugewandt ist. Solche Vertiefungen können z.B. durch Ätzen in der zweiten Glasplatte erzeugt sein, z.B. nach Bestrahlung der zweiten Glasplatte entlang des Wegs solcher Vertiefungen, z.B. mit nebeneinander eingestrahlten gepulsten Laserstrahlen vor dem Ätzen.

Die in der ersten Glasplatte gebildeten Ausnehmungen können in einem oberen Dickenabschnitt einen größeren Querschnitt aufweisen, als in einem daran angrenzenden unteren Dickenabschnitt, der in zumindest zwei Teilausnehmungen unterteilt ist, wobei der untere Dickenabschnitt an die zweite Platte angrenzt, z.B. mit dieser unmittelbar oder mittels einer Schicht aus geschmolzener und erstarrter Glasfritte verbunden ist. In dieser Ausführungsform werden die endständigen Querschnittsöffnungen der zumindest zwei Teilausnehmungen, die an die zweite Platte angrenzen, von der zweiten Platte überdeckt. Die Ausnehmungen, die sich über den oberen Dickenabschnitt erstrecken, überdecken zumindest zwei Teilausnehmungen. Zwischen den Teilausnehmungen, die sich über den unteren Dickenabschnitt erstrecken, sind einstückig aus der ersten Glasplatte über den unteren Dickenabschnitt gebildete Teilwände angeordnet. Diese Teilwände sind voneinander um die Teilausnehmungen beabstandet. Diese Teilwände werden durch Einstrahlen von Laserpulsen, die im Bereich der Teilwände nur maximal in den oberen Dickenabschnitt der ersten Glasplatte eindringen, und Einstrahlen von Laserpulsen im Bereich der Teilausnehmungen, die die erste Glasplatte vollständig durchdringen, und anschließendes Ätzen der ersten Glasplatte erzeugt. Beim Ätzen bildet sich die im oberen Dickenabschnitt, der an die erste Oberfläche der ersten Glasplatte angrenzt, eine Ausnehmung, die sich über den Bereich von zumindest zwei Teilausnehmungen erstreckt. Die Längsmittelachsen der Teilausnehmungen können z.B. in einem Abstand von 10 bis 100 µm angeordnet sein. Die Teilwände erstrecken sich über den unteren Dickenabschnitt zwischen den Teilausnehmungen, wobei die Teilausnehmungen in der Ebene der an die zweite Platte angrenzenden zweiten Oberfläche der ersten Glasplatte z.B. einen Durchmesser von 1 bis 50 aufweisen können. Der obere Dickenabschnitt wird vorliegend auch als erster Dickenabschnitt bezeichnet, der untere Dickenabschnitt wird auch als zweiter Dickenabschnitt bezeichnet. Der obere Dickenabschnitt und der untere Dickenabschnitt erstrecken sich ausgehend von der ersten Oberfläche der ersten Glasplatte, unabhängig voneinander z.B. bis in zumindest 20 % oder zumindest 30% oder zumindest 40 oder zumindest 50 %, z.B. bis zu 80 % oder bis zu 70 % oder bis zu 60% der Dicke der ursprünglichen ersten Glasplatte. Dabei erstreckt sich der untere Dickenabschnitt in größerem Ausmaß in die Dicke der ersten Glasplatte als der obere Dickenabschnitt. Generell erstrecken sich die Dickenabschnitte nicht über die ganze Dicke der ersten Glasplatte. Z.B. können der erste und insbesondere der zweite Dickenabschnitt so vorbestimmt sein, dass nach dem Ätzen im Bereich der Ausnehmung eine Dicke der ersten Glasplatte, bzw. zwischen der Ausnehmung und der zweiten Oberfläche, von zumindest 5%, zumindest 10 % oder zumindest 15 % oder zumindest 20 % bestehen bleibt.

Auf der der zweiten Platte gegenüberliegenden ersten Oberfläche der ersten Glasplatte kann eine dritte Platte angeordnet und mit der ersten Glasplatte verbunden sein. Die dritte Platte weist dritte Ausnehmungen auf, die sich durch die vollständige Dicke der dritten Platte erstrecken und passend, insbesondere fluchtend, über den Ausnehmungen der ersten Glasplatte angeordnet sind. Dabei können die dritten Ausnehmungen einen Durchmesser aufweisen, der gleich oder größer als der Durchmesser der Ausnehmungen der ersten Glasplatte ist. Optional können sich die dritten Ausnehmungen über jeweils zwei oder mehr Ausnehmungen der ersten Glasplatte erstrecken. Die dritten Ausnehmungen sind z.B. zur Verwendung als Einfülltrichter zum Befüllen der Ausnehmungen in der ersten Glasplatte geeignet. Die dritten Ausnehmungen können z.B. innerhalb der dritten Platte ein Volumen von 0,01 bis 10 µL, z.B. 0,1 bis 3 µL aufweisen.

Generell können die Ausnehmungen in der ersten Glasplatte jeweils in einem Abstand ihrer Längsmittelachsen von 20 bis 200 µm angeordnet sein. Bei der Herstellung werden generell die durch die erste Glasplatte durchgehenden Laserpulse in dem Abstand der Längsmittelachsen der Ausnehmungen bzw. Teilausnehmungen eingestrahlt. Die Ausnehmungen in der ersten Glasplatte können in der Ebene der zweiten Oberfläche der ersten Glasplatte z.B. einen Durchmesser von 5 bis 200 µm aufweisen.

Die dritte Platte kann eine Glasplatte sein, z.B. einer Dicke von zumindest 100 µm oder zumindest 300 µm, z.B. bis 2000 µm oder bis 1000 µm. Die dritten Ausnehmungen können einen Innendurchmesser von z.B. zumindest 0,2 mm oder zumindest 0,5 mm aufweisen, z.B. bis 3 mm oder bis 2 mm oder bis 1 mm. Die dritte Platte kann mittels anodischem Bonding, mittels einer geschmolzenen und erstarrten Schicht aus Glasfritte zwischen erster Glasplatte und dritter Platte, z.B. mittels Siebdruck aufgetragen, oder mittels Fusion-Bonding mit der ersten Glasplatte verbunden werden.

Optional kann die zweite Platte, insbesondere in der Ausführungsform einer zweiten Glasplatte, in dem Bereich, in dem sie die in der ersten Glasplatte gebildeten Ausnehmungen überdeckt, zweite Ausnehmungen aufweisen, die einen kleineren Durchmesser haben als die Ausnehmungen der ersten Glasplatte. Zweite Ausnehmungen können zur ersten Glasplatte offene Sacklöcher sein oder sich durch die vollständige Dicke der zweiten Platte erstrecken, so dass sie eine Rückhalteeinrichtung für größere Partikel bilden. Zweite Ausnehmungen können in der zweiten Platte durch die Verfahren gebildet werden, die mit Bezug auf die Ausbildung von Ausnehmungen in der ersten Glasplatte beschrieben sind. Die zweiten Ausnehmungen können kegelförmig ausgebildet sein, wobei bevorzugt ihr kleinerer Durchmesser in der Ebene der Oberfläche der zweiten Platte liegt, die der ersten Glasplatte zugewandt und mit dieser verbunden ist. Die zweiten Ausnehmungen können z.B. Durchmesser von 1 µm bis 50 µm in der Ebene der der ersten Glasplatte zugewandten Oberfläche der zweiten Platte aufweisen. Bevorzugt weisen die Ausnehmungen der zweiten Platte einen Durchmesser auf, der um einen Faktor 5 bis 10 kleiner ist als der Durchmesser einer Zelle, die in dem Reaktionsgefäß enthalten ist, z.B. Ausnehmungen mit einem Durchmesser von 1 bis 3 µm, insbesondere von 1 oder von 1,5 µm bis 2 µm, z.B. für einen Zelldurchmesser von 7 bis 15 µm. Z.B. können die Längsmittelachsen der zweiten Ausnehmungen in einem Abstand von 10 bis 100 µm angeordnet sein. Eine zweite Platte, die bevorzugt eine zweite Glasplatte ist, hat bevorzugt eine Dicke im Bereich von 50 bis 500 µm, z.B. bis 150 µm oder bis 100 µm.

In dieser Ausführungsform kann der Boden der Ausnehmungen der zweiten Glasplatte Mikrostrukturen aufweisen, die für eine Nahfeldbeleuchtung des Innenvolumens der Ausnehmungen eingerichtet sind. Solche Mikrostrukturen können z.B. die Form von schmalen, hohen Glasspitzen haben und so als Lichtwellenleiter für die Beleuchtung fungieren oder einzelne Zellen bzw. Ansammlungen von Zellen in ihrer Lage oder Position beeinflussen können. Solche Strukturen können hergestellt werden, indem z.B. eine Ausnehmung durch das Aufätzen einer Vielzahl von Positionen, an denen eng beabstandet Laserpulse eingestrahlt wurden, gebildet wird. Wird in der Mitte der Ausnehmung ein einzelner Laserpuls ausgelassen, so bleibt nach dem Ätzprozess hier eine Glasspitze innerhalb der Ausnehmung stehen. Generell kann im Verfahren eine Ausnehmung durch Einstrahlen von Laserpulsen nebeneinander an Positionen und anschließendes Ätzen erzeugt werden, wobei die Positionen in jeweils gleichen Abständen voneinander von maximal 10 µm, z.B. 1 bis 5 µm oder bis 3 µm angeordnet sind und zusammen eine Stelle bilden, wobei zumindest 2 oder zumindest 3 Positionen in einem größeren Abstand angeordnet sind, z.B. in einem Abstand von 10 bis 30 µm, z.B. 15 bis 20 µm Abstand. Die zumindest 2 oder 3 Positionen der Laserpulse, die in einem größeren Abstand angeordnet sind, weisen zwischen sich z.B. den Bereich auf, in dem ein Laserpuls im gleichen Abstand ausgelassen ist, bzw. umgeben den Bereich, in dem beim Ätzen eine Glasspitze stehenbleibt.

In Ausführungsformen zur Herstellung von Reaktionsgefäßen, die in einer zweiten Glasplatte Ausnehmungen aufweisen, wird das Ätzen der zweiten Glasplatte z.B. für eine Zeitdauer durchgeführt, die zum Erreichen einer gewünschten Tiefe der Ausnehmungen im Glasvolumen der zweiten Glasplatte ausreicht, die in einem Abstand von der zweiten Oberfläche liegt, bzw. nur für eine Zeitdauer geätzt wird, nach der die zweite Glasplatte noch eine geschlossene zweite Oberfläche aufweist. Optional kann dabei der Boden der Reaktionsgefäße konkave Vertiefungen aufweisen, die einen parabel- oder kegelförmigen Querschnitt aufweisen. Solche konkaven Vertiefungen können an jeder Position gebildet werden, an der ein Laserstrahl punktförmig eingestrahlt wurde. Bevorzugt weisen solche konkaven Vertiefungen eine Querschnittsöffnung und eine Tiefe von wenigen Mikrometern, z.B. 1 bis 5 µm auf.

Die Ausführungsform, in der die zweite Platte zweite Ausnehmungen aufweist, ist insbesondere zur Verwendung der Ausnehmungen in der ersten Platte als Kulturgefäße für Zellen, insbesondere menschliche bzw. tierische Zellen, geeignet. Bei einem Verfahren zur Kultivierung von Zellen in den Ausnehmungen kann eine Flüssigkeit, z.B. Kultivierungsmedium und/oder zu testende Reagenzien, durch die zweiten Ausnehmungen bewegt werden, z.B. in die Ausnehmungen der ersten Glasplatte eingebracht oder daraus abgeführt werden. Z.B. kann eine Zellsuspension in Medium aus einer Richtung gegenüber der zweiten Platte bzw. in die offenen Querschnittsöffnungen der Ausnehmungen in die Ausnehmungen der ersten Glasplatte gefüllt werden, wobei Medium durch die zweiten Ausnehmungen austritt. Die Zellsuspension in Medium kann z.B. Vollblut sein, oder kultivierte Zellen in Puffer oder Kulturmedium. Diese Ausführungsform ist zur Verwendung als Filter, z.B. für eine Zellsuspension in Medium geeignet. Dabei kann im Verfahren ein Medium, z.B. Kulturmedium oder Puffer, die freie Oberfläche der zweiten Platte kontaktieren oder an dieser entlang strömen gelassen werden, um einen Medienwechsel durch die zweiten Ausnehmungen zu erzeugen, z.B. um Stoffwechselprodukte abzuführen, die durch die zweiten Ausnehmungen treten.

Weiter betrifft die Erfindung ein Verfahren zur Analyse, bei dem die Reaktionsgefäße in Glas mit Licht bestrahlt werden und von den Reaktionsgefäßen ausgehendes Licht detektiert wird, sowie die Verwendung der Reaktionsgefäße aus Glas in dem Analyseverfahren.

Dabei kann Licht zur Analyse bevorzugt etwa senkrecht auf die zweite Platte eingestrahlt werden, auf die Oberfläche der zweiten Glasplatte, die der ersten Glasplatte gegenüberliegt, oder auf die Oberfläche der zweiten Glasplatte, die der ersten Glasplatte zugewandt ist.

Es hat sich gezeigt, dass Ausnehmungen, insbesondere Ausnehmungen, die sich kegelförmig von der ersten Oberfläche in das Volumen der ersten Glasplatte erstrecken, bei Bestrahlung mit z.B. sichtbarem Licht mit ihrer Wandung einen deutlichen Kontrast zum Boden der Ausnehmungen bilden. Bei optischer Detektion kann daher der Umfang der Ausnehmungen aufgenommen und dargestellt werden, insbesondere als dunkler Ring im Kontrast zum Boden der Ausnehmungen.

Weiter betrifft die Erfindung Verfahren zur Analyse mit dem Schritt des Bereitstellens von Reaktionsgefäßen, die nach einem erfindungsgemäßen Herstellungsverfahren hergestellt sind, oder die erfindungsgemäße Reaktionsgefäße sind, dem Einbringen von Probe, z.B. Patientenprobe, die zellfrei, z.B. Blutplasma, oder zellhaltig, z.B. Vollblut oder aus Vollblut abgetrennte Zellen, oder Gewebematerial sein kann, in Reaktionsgefäße, vorher, gleichzeitig oder anschließend, Zugeben zumindest eines Reagenzes in Reaktionsgefäße, und Analysieren.

Optional kann das zumindest eine Reagenz, einfach oder nacheinander mehrfach, in unterschiedlichen Mengen in mehrere Reaktionsgefäße zugegeben werden. Das Reagenz kann z.B. ein pharmazeutischer Wirkstoff sein, und das Analysieren kann die Messung der Wirkung des Wirkstoffs auf die Probe umfassen. Optional kann das Verfahren einen oder mehrere Inkubationsschritte umfassen, z.B. unter Zellkulturbedingungen (37°C, 5% CO₂-Atmosphäre, ruhend oder mit Bewegung).

Das Analysieren kann eine optische Messung der Reaktionsgefäße sein, z.B. während oder nach Sequenzierung von DNA und/oder RNA und/oder von Protein in den Reaktionsgefäßen nach Zugabe von Reagenzien zur Sequenzierung, optional nach Lyse von Zellen, oder die Bestimmung von Proteinen, z.B. nach Reaktion mit einem als Reagenz zugegebenen Bindemolekül, z.B. einem Antikörper, der bevorzugt mit einem Farbstoff markiert ist. Bevorzugt umfasst das Analysieren z.B. bei zellhaltigen Proben die Bestimmung der transkribierten RNAs und/oder der translatierten Proteine, insbesondere für Proben, denen kein Wirkstoff zugegeben wurde im Vergleich mit Proben, denen Wirkstoff zugegeben wurde. Optional kann das Verfahren zur Analyse die Entnahme eines Anteils der Probe aus einem Reaktionsgefäß umfassen, weiter optional das Einfüllen des entnommenen Probeanteils in ein weiteres erfindungsgemäßes oder erfindungsgemäß hergestelltes Reaktionsgefäß.

Das Zugeben von Probe und/oder von Reagenz in die Reaktionsgefäße kann z.B. durch Bewegen von Flüssigkeitstropfen der Probe und/oder von Flüssigkeitstropfen des Reagenzes erfolgen, wobei die Flüssigkeitstropfen z.B. als Teil eines Flüssigkeitsstrahls erzeugt und bewegt werden, wie dies z.B. durch Einwirken elektromagnetischer Strahlung, durch Beaufschlagung mit Schall oder Ultraschall, durch Anlegen eines elektrischen Felds oder durch Druckbeaufschlagung erfolgen. Das Erzeugen von Flüssigkeitstropfen ist als Tintenstrahldruckverfahren oder Pipettieren bekannt. Alternativ können laserbasierte Druckverfahren, z.B. Laser-Transfer-Druck, eingesetzt werden. Bevorzugt erfolgt das Zugeben von Probe und/oder von Reagenz ohne Kontakt der Dosiervorrichtung mit dem Reaktionsgefäß.

In Ausführungsformen, in denen Leiterbahnen an den Ausnehmungen angeordnet sind, kann bei Verfahren zur Analyse das Innenvolumen der Ausnehmungen mit Spannung beaufschlagt werden und zwischen den Leiterbahnen können elektrische Parameter gemessen werden, die im Innenvolumen vorliegen.

Die Figuren zeigen schematisch in
- Fig. 1 im Querschnitt senkrecht zur Oberfläche der ersten Glasplatte eine Ausführungsform der Reaktionsgefäße in Glas aus zwei Glasplatten,
- Fig. 2 in Aufsicht auf die erste Oberfläche der ersten Glasplatte die optische Analyse eines erfindungsgemäß hergestellten Reaktionsgefäßes,
- Fig. 3 a) - b) mehrteilige Ausführungsformen im Querschnitt senkrecht zur Oberfläche der ersten Glasplatte und in
- Fig. 4 eine weitere Ausführungsform im Querschnitt senkrecht zur Oberfläche der ersten Glasplatte.

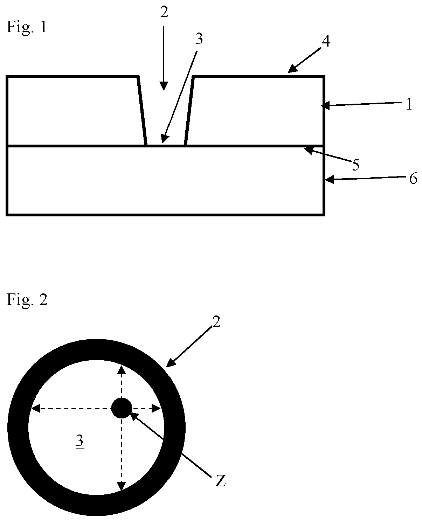

Die Fig. 1 zeigt eine erste Glasplatte 1, in der sich die Ausnehmung 2 durch die vollständige Dicke erstreckt und der Boden 3 durch eine zweite Glasplatte 6 gebildet wird, die dicht mit der zweiten Oberfläche 5 der ersten Glasplatte 1 verbunden ist.

Die Fig. 2 zeigt in Aufsicht auf eine erste Glasplatte 1 eine Ausnehmung 2, deren Wandung einen starken optischen Kontrast zum Boden 3 bildet, so dass die Wandung als umfängliche Begrenzung des Bodens 3 deutlich dargestellt wird. Ein Partikel, z.B. eine biologische Zelle Z, kann bei einer durch den Boden 3 gerichteten Beleuchtung mit gutem Kontrast gegen den Boden 3 gesehen werden, insbesondere bei Markierung der Zelle Z durch einen Farbstoff, z.B. einen Fluoreszenzfarbstoff.

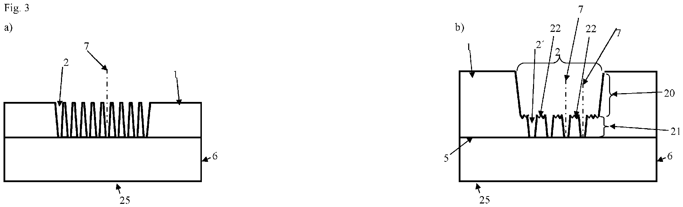

Die Fig. 3 a) zeigt die Anordnung von zumindest zwei, vorliegend dargestellt zehn, Ausnehmungen 2, deren Längsmittelachsen 7 in gleichem Abstand voneinander angeordnet sind. Die Ausnehmungen 2 sind von Wandungen eingefasst, die sich durch die vollständige Dicke der ersten Glasplatte 1 erstrecken. Dabei laufen die Ausnehmungen 2 kegelförmig von der ersten Oberfläche 4 zur zweiten Oberfläche 5 der ersten Glasplatte 1 zu. Die Querschnittsöffnungen der Ausnehmungen 2 werden in der Ebene der zweiten Oberfläche 5 von der zweiten Platte 6 überdeckt.

Die Fig. 3 b) zeigt eine Ausführungsform, in der in der ersten Glasplatte 1 eine Ausnehmung durch Ätzen erzeugt ist, nachdem die erste Glasplatte 1 an den Stellen, an denen Teilausnehmungen 2' gebildet werden, mit durch ihre vollständige Dicke durchgehenden Laserpulsen bestrahlt wurde und dazwischen mit Laserpulsen bestrahlt wurde, die sich maximal über einen oberen Dickenabschnitt 20 erstrecken. Der obere Dickenabschnitt 20 erstreckt sich von der ersten Oberfläche 4 der ersten Glasplatte 1 bis angrenzend an die Teilausnehmungen 2', wobei zwischen den Teilausnehmungen 2' an den Orten, an denen Laserpulse maximal bis in die Tiefe des oberen Dickenabschnitts 20 eingestrahlt wurden, Teilwände 22 zurückbleiben. Die endständigen Querschnittsöffnungen der Teilausnehmungen 2', die dem ersten Dickenabschnitt 20 gegenüberliegen, sind von einer zweiten Platte 6 überdeckt, die mit der ersten Platte 1 verbunden ist.

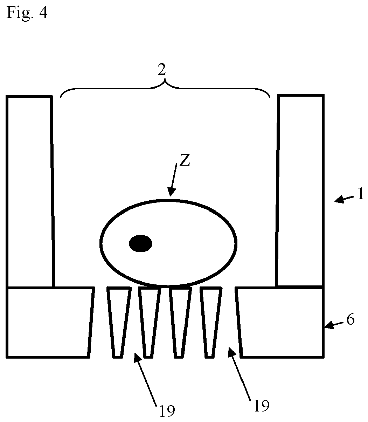

Die Fig. 4 zeigt eine Ausführungsform, bei der die zweite Platte 6 zweite Ausnehmungen 19 im Bereich der Ausnehmungen 2 aufweist, die in der ersten Glasplatte 1 gebildet sind. Die zweiten Ausnehmungen 19 können sich optional nur bis in einen Anteil der Dicke der zweiten Platte 6 erstrecken, oder sich durch die vollständige Dicke der zweiten Platte 6 erstrecken, wie hier dargestellt. Die zweiten Ausnehmungen weisen in der Ebene der Oberfläche der zweiten Platte 6, die mit der ersten Glasplatte 1 verbunden ist, Durchmesser auf, der z.B. um den Faktor 5 bis 10 kleiner ist als der Durchmesser einer Zelle Z. Eine zweite Platte 6 mit durch ihre vollständige Dicke durchgehenden zweiten Ausnehmungen 19 bilden eine Rückhaltevorrichtung für größere Partikel, z.B. eine Zelle Z.

Entsprechend einer bevorzugten Ausführungsform laufen in Fig. 4 die zweiten Ausnehmungen 19 kegelförmig zu und weisen ihren kleineren Durchmesser in der Ebene der der ersten Glasplatte zugewandten Oberfläche der zweiten Platte 6 auf.

### Bezugszeichenliste:

| | | | |
|---|---|---|---|
| 1 | erste Glasplatte | 7 | Längsmittelachse |
| 2 | Ausnehmung | 20 | oberer, erster Dickenabschnitt |
| 2' | Teilausnehmung | 21 | unterer, zweiter Dickenabschnitt |
| 3 | Boden | 22 | Teilwände |
| 4 | erste Oberfläche | 24 | erste Oberfläche der zweiten Platte |
| 5 | zweite Oberfläche | 25 | zweite Oberfläche der zweiten Platte |
| 6 | zweite Platte | Z | Zelle |

## Patentansprüche

1. Verfahren zur Herstellung einer Vielzahl von Reaktionsgefäßen aus Glas, die als Ausnehmungen (2) in einer ersten Glasplatte (1) gebildet sind, mit den Schritten
1. Einstrahlen von Laserpulsen einer Wellenlänge, für die die erste Glasplatte (1) durchlässig ist, auf die Stellen einer ersten Oberfläche (4) der ersten Glasplatte (1), an denen jeweils eine Ausnehmung (2) als Reaktionsgefäß erzeugt werden soll, wobei Laserstrahlen punktförmig in der Ebene der ersten Oberfläche (4) in einem Abstand von genau dem Durchmesser eines der Reaktionsgefäße zuzüglich der Dicke einer Wand zwischen den Reaktionsgefäßen eingestrahlt werden,
2. in Abwesenheit einer Beschichtung aus Ätzresist, Ätzen der ersten Glasplatte (1) für eine Zeitdauer, die zum Erzeugen von Ausnehmungen (2) entlang der Stellen ausreicht, wobei das Ätzen bis zur Ausbildung von Ausnehmungen (2) mit einer Tiefe, die sich über die vollständige Dicke der ersten Glasplatte (1) erstreckt, erfolgt, und Verbinden einer zweiten Platte (6), die aus Glas besteht, mit der der ersten Oberfläche (4) gegenüberliegenden zweiten Oberfläche (5) der ersten Glasplatte (1),
wobei das Verbinden durch anodisches Bonding oder allein durch Anordnen der ersten Glasplatte (1) gegen die zweite Platte (6) mit anschließendem Erwärmen erfolgt.

2. Verfahren nach Anspruch 1, **dadurch gekennzeichnet, dass** die Tiefe der Ausnehmungen (2) mindestens 30 µm beträgt.

3. Verfahren nach einem der voranstehenden Ansprüche, **dadurch gekennzeichnet, dass** die Ausnehmungen (2) ein Streckungsverhältnis von zumindest 2 von Tiefe zu Durchmesser, der in der Ebene der ersten Oberfläche (4) gemessen ist, aufweisen

4. Verfahren nach einem der voranstehenden Ansprüche, **dadurch gekennzeichnet, dass** beim Verbinden durch anodisches Bonding die der ersten Glasplatte (1) zugewandte Oberfläche der zweiten Glasplatte (6) und/oder die zweite Oberfläche (5) der ersten Glasplatte (1) mit Silizium beschichtet wird.

5. Verfahren nach einem der voranstehenden Ansprüche, **dadurch gekennzeichnet, dass** die erste Glasplatte (1) an den Stellen, an denen eine Ausnehmung (2) erzeugt werden soll, an mehreren voneinander beabstandeten Positionen mit Laserpulsen bestrahlt wird.

6. Verfahren nach Anspruch 5, **dadurch gekennzeichnet, dass** an einem Teil der Positionen (12) Laserpulse weniger tief bis in einen ersten Dickenabschnitt (20) in die erste Glasplatte (1) eingestrahlt werden, und an einem Teil der Positionen (13) Laserpulse tiefer bis in einen angrenzenden zweiten Dickenabschnitt (21) in die erste Glasplatte (1) eingestrahlt werden, wobei beim Ätzen eine Ausnehmung (2), die sich über den ersten Dickenabschnitt erstreckt, gebildet wird und von Teilwänden (22) beabstandete Teilausnehmungen (2'), die sich über den zweiten Dickenabschnitt (21) erstrecken, gebildet werden.

7. Verfahren nach einem der voranstehenden Ansprüche , **dadurch gekennzeichnet, dass** die Positionen in einem Abstand von maximal 10 µm angeordnet sind, wobei zumindest drei Positionen zumindest 20 µm voneinander beabstandet sind.

8. Verfahren nach einem der voranstehenden Ansprüche, **dadurch gekennzeichnet, dass** die Fokuslage der Laserstrahlung und/oder der Laserpulse so eingestellt wird, dass die Laserstrahlung und/oder der Laserpulse nur bis in einen Dickenabschnitt in die erste Glasplatte (1) eindringen, der sich nicht über die ganze Dicke der ersten Glasplatte erstreckt (1).

9. Verfahren nach einem der voranstehenden Ansprüche, **dadurch gekennzeichnet, dass** an den Stellen der ersten Glasplatte (1), an denen eine Ausnehmung (2) erzeugt werden soll, ein Laserstrahl auf einem umfänglich geschlossenen Weg eingestrahlt wird.

10. Verfahren nach Anspruch 14, **dadurch gekennzeichnet, dass** der Laserstrahl, der auf einem umfänglich geschlossenen Weg eingestrahlt wird, durch nebeneinander eingestrahlte Laserstrahlpulse gebildet wird.

11. Verfahren nach einem der voranstehenden Ansprüche, **dadurch gekennzeichnet, dass** in der zweiten Platte (6) zweite Ausnehmungen (19) erzeugt werden und die zweite Platte (6) mit ihren zweiten Ausnehmungen (19) angrenzend an die Ausnehmungen (2) der ersten Glasplatte (1) mit der ersten Glasplatte (1) verbunden wird.

12. Reaktionsgefäße, die als Ausnehmungen aus Glas gebildet sind, erhältlich nach einem Verfahren nach einem der voranstehenden Ansprüche, mit einer Tiefe der Ausnehmungen von zumindest 30 µm in einer ersten Glasplatte (1) und einem Streckungsverhältnis von zumindest 2 von Tiefe zu Durchmesser, der in der Ebene einer ersten Oberfläche (4) der ersten Glasplatte (1) gemessen ist, wobei die Ausnehmungen (2) von Wandungen eingefasst sind, deren Stirnflächen in einer gemeinsamen Ebene angeordnet sind und die erste Oberfläche (4) der ersten Glasplatte (1) bilden, von der die Ausnehmungen (2) ausgenommen sind, wobei sich die Ausnehmungen (2) durch die vollständige Dicke der ersten Glasplatte (1) erstrecken und eine zweite Platte (6), die den Boden (3) der Reaktionsgefäße bildet, durch anodisches Bonding oder allein durch Anordnen der ersten Glasplatte (1) gegen die zweite Platte (6) mit anschließendem Erwärmen mit einer Oberfläche (4, 5) der ersten Glasplatte (1) verbunden ist.

13. Reaktionsgefäße nach Anspruch 12, **dadurch gekennzeichnet, dass** die Leiterbahnen innerhalb von Gräben angeordnet sind, die in der Oberfläche der zweiten Platte (6) erzeugt sind, die mit der ersten Glasplatte (1) verbunden ist.

14. Verfahren zur Analyse einer Probe mit dem Schritt des Bereitstellens von Reaktionsgefäßen, die nach einem Verfahren nach einem der Ansprüche 1 bis 11 hergestellt sind oder die Reaktionsgefäße nach einem der Ansprüche 12 bis 13 sind, mit dem Einbringen von Probe, die zellfrei oder zellhaltig ist, in die Reaktionsgefäße, Zugeben zumindest eines Reagenzes in Reaktionsgefäße, und dem optischen Messen der Reaktionsgefäße.

## Claims

1. Method of manufacturing a plurality of glass reaction vessels formed as recesses (2) in a first glass plate (1), comprising the steps of
1. Irradiating of laser pulses of a wavelength, for which the first glass plate (1) is transparent, onto the locations of a first surface (4) of the first glass plate (1) at which locations a recess (2) is to be produced as a reaction vessel in each case, laser beams being irradiated in a point-like manner in the plane of the first surface (4) at a distance of exactly the diameter of one of the reaction vessels plus the thickness of a wall between the reaction vessels,
2. in the absence of a coating of etch resist, etching the first glass plate (1) for a period of time sufficient to produce recesses (2) along the locations, the etching being carried out until recesses (2) are formed with a depth extending over the entire thickness of the first glass plate (1), and bonding a second plate (6), which is made of glass, to the second surface (5) of the first glass plate (1) opposite the first surface (4),
wherein the bonding is carried out by anodic bonding or solely by placing the first glass plate (1) against the second plate (6) with subsequent heating.

2. Method according to claim 1, **characterised in that** the depth of the recesses (2) is at least 30 µm.

3. Method according to one of the preceding claims, **characterised in that** the recesses (2) have an aspect ratio of at least 2 of depth to diameter measured in the plane of the first surface (4).

4. Method according to one of the preceding claims, **characterised in that**, during bonding by anodic bonding, the surface of the second glass plate (6) facing the first glass plate (1) and/or the second surface (5) of the first glass plate (1) is coated with silicon.

5. Method according to one of the preceding claims, **characterised in that** the first glass plate (1) is irradiated with laser pulses at a plurality of spaced-apart positions at the locations at which a recess (2) is to be produced.

6. Method according to claim 5, **characterised in that** at some of the positions (12) laser pulses are irradiated less deeply into the first glass plate (1) in a first thickness section (20), and at some of the positions (13) laser pulses are irradiated more deeply into the first glass plate (1) in an adjacent second thickness section (21), wherein a recess (2) extending over the first thickness section is formed during etching and partial recesses (2') spaced apart by partial walls (22) and extending over the second thickness section (21) are formed.

7. Method according to one of the preceding claims, **characterised in that** the positions are arranged at a maximum distance of 10 µm, with at least three positions being at least 20 µm apart.

8. Method according to one of the preceding claims, **characterised in that** the focal position of the laser radiation and/or of the laser pulses is set such that the laser radiation and/or the laser pulses only penetrate into the first glass plate (1) up to a thickness section which does not extend over the entire thickness of the first glass plate (1).

9. Method according to one of the preceding claims, **characterised in that** a laser beam is irradiated along a circumferentially closed path at the locations of the first glass plate (1) at which a recess (2) is to be produced.

10. Method according to claim 14, **characterised in that** the laser beam, which is irradiated on a circumferentially closed path, is formed by laser beam pulses irradiated side by side.

11. Method according to one of the preceding claims, **characterised in that** second recesses (19) are produced in the second plate (6) and the second plate (6) is connected to the first glass plate (1) with its second recesses (19) adjacent to the recesses (2) of the first glass plate (1).

12. Reaction vessels, which are formed as recesses of glass, obtainable by a method according to one of the preceding claims, with a depth of the recesses of at least 30 µm in a first glass plate (1) and an aspect ratio of at least 2 of depth to diameter, which is measured in the plane of a first surface (4) of the first glass plate (1), the recesses (2) being enclosed by walls the end faces of which are arranged in a common plane and form the first surface (4) of the first glass plate (1), from which the recesses (2) are recessed, the recesses (2) extending through the entire thickness of the first glass plate (1) and a second plate (6), which forms the base (3) of the reaction vessels, being bonded to a surface (4, 5) of the first glass plate (1) by anodic bonding or solely by arranging the first glass plate (1) against the second plate (6) with subsequent heating.

13. Reaction vessels according to claim 12, **characterised in that** the conductive tracks are arranged within trenches created in the surface of the second plate (6), which is connected to the first glass plate (1).

14. Method for analysing a sample comprising the step of providing reaction vessels which are produced by a method according to any one of claims 1 to 11 or which are reaction vessels according to any one of claims 12 to 13, with the introduction of sample, which is cell-free or cell-containing, into the reaction vessels, adding at least one reagent into reaction vessels, and optically measuring the reaction vessels.

## Revendications

1. Procédé de fabrication d'une pluralité de récipients de réaction en verre, qui sont formés comme des évidements (2) dans une première plaque de verre (1), avec les étapes suivantes
1. l'irradiation d'impulsions laser d'une longueur d'onde pour laquelle la première plaque de verre (1) est transparente, sur les endroits d'une première surface (4) de la première plaque de verre (1) où un évidement (2) doit respectivement être produit comme récipient de réaction, des rayons laser étant irradiés ponctuellement dans le plan de la première surface (4) à une distance correspondant exactement au diamètre de l'un des récipients de réaction plus l'épaisseur d'une paroi entre les récipients de réaction,
2. en l'absence d'un revêtement de résist à la gravure, la gravure de la première plaque de verre (1) pendant une durée suffisante pour créer des évidements (2) le long desdits emplacements, la gravure étant effectuée jusqu'à la formation d'évidements (2) ayant une profondeur s'étendant sur toute l'épaisseur de la première plaque de verre (1), et la liaison d'une seconde plaque (6) constituée de verre à la seconde surface (5) de la première plaque de verre (1) opposée à la première surface (4),
l'assemblage étant réalisé par liaison anodique ou uniquement par mise en place de la première plaque de verre (1) contre la deuxième plaque (6), suivie d'un chauffage.

2. Procédé selon la revendication 1, **caractérisé en ce que** la profondeur des évidements (2) est d'au moins 30 µm.

3. Procédé selon l'une quelconque des revendications précédentes, **caractérisé en ce que** les évidements (2) ont un rapport d'allongement d'au moins 2 de la profondeur au diamètre mesuré dans le plan de la première surface (4).

4. Procédé selon l'une des revendications précédentes, **caractérisé en ce que**, lors de l'assemblage par liaison anodique, la surface de la deuxième plaque de verre (6) tournée vers la première plaque de verre (1) et/ou la deuxième surface (5) de la première plaque de verre (1) est revêtue de silicium.

5. Procédé selon l'une quelconque des revendications précédentes, **caractérisé en ce que** la première plaque de verre (1) est irradiée par des impulsions laser en plusieurs positions espacées les unes des autres, aux endroits où un évidement (2) doit être créé.

6. Procédé selon la revendication 5, **caractérisé en ce que** dans une partie des positions (12), des impulsions laser sont irradiées moins profondément jusque dans une première section d'épaisseur (20) dans la première plaque de verre (1), et dans une partie des positions (13), des impulsions laser sont irradiées plus profondément jusque dans une deuxième section d'épaisseur (21) adjacente dans la première plaque de verre (1), un évidement (2), qui s'étend sur la première section d'épaisseur, étant formé lors de la gravure et des évidements partiels (2') espacés par des parois partielles (22), qui s'étendent sur la deuxième section d'épaisseur (21), étant formés.

7. Procédé selon l'une des revendications précédentes, **caractérisé en ce que** les positions sont disposées à une distance maximale de 10 µm, au moins trois positions étant espacées d'au moins 20 µm.

8. Procédé selon l'une des revendications précédentes, **caractérisé en ce que** la position focale du rayonnement laser et/ou des impulsions laser est réglée de telle sorte que le rayonnement laser et/ou les impulsions laser ne pénètrent dans la première plaque de verre (1) que jusqu'à une section d'épaisseur qui ne s'étend pas sur toute l'épaisseur de la première plaque de verre (1).

9. Procédé selon l'une quelconque des revendications précédentes, **caractérisé en ce que**, aux endroits de la première plaque de verre (1) où un évidement (2) doit être créé, un faisceau laser est irradié selon un trajet fermé sur sa circonférence.

10. Procédé selon la revendication 14, **caractérisé en ce que** le faisceau laser, qui est irradié sur un trajet fermé sur sa circonférence, est formé par des impulsions de faisceau laser irradiées côte à côte.

11. Procédé selon l'une quelconque des revendications précédentes, **caractérisé en ce que** des deuxièmes évidements (19) sont créés dans la deuxième plaque (6) et **en ce que** la deuxième plaque (6) est assemblée à la première plaque de verre (1) par ses deuxièmes évidements (19) adjacents aux évidements (2) de la première plaque de verre (1).

12. Récipients de réaction formés d'évidements en verre, pouvant être obtenus par un procédé selon l'une des revendications précédentes, avec une profondeur des évidements d'au moins 30 µm dans une première plaque de verre (1) et un rapport d'étirage d'au moins 2 de la profondeur au diamètre, qui est mesuré dans le plan d'une première surface (4) de la première plaque de verre (1), les évidements (2) étant entourés de parois, dont les faces frontales sont disposées dans un plan commun et forment la première surface (4) de la première plaque de verre (1), de laquelle les évidements (2) sont exclus, les évidements (2) s'étendant à travers l'épaisseur complète de la première plaque de verre (1) et une deuxième plaque (6), qui forme le fond (3) des récipients de réaction, étant reliée à une surface (4, 5) de la première plaque de verre (1) par liaison anodique ou uniquement en plaçant la première plaque de verre (1) contre la deuxième plaque (6) avec chauffage ultérieur.

13. Récipients de réaction selon la revendication 12, **caractérisés en ce que** les pistes conductrices sont disposées à l'intérieur de tranchées créées dans la surface de la deuxième plaque (6) qui est reliée à la première plaque de verre (1).

14. Procédé d'analyse d'un échantillon comprenant l'étape consistant à fournir des récipients de réaction qui sont préparés selon un procédé selon l'une quelconque des revendications 1 à 11 ou qui sont des récipients de réaction selon l'une quelconque des revendications 12 à 13, comprenant l'introduction d'un échantillon qui est exempt de cellules ou qui contient des cellules dans les récipients de réaction, l'addition d'au moins un réactif dans des récipients de réaction, et la mesure optique des récipients de réaction.
